(19) Europäisches Patentamt
European Patent Office
Office européen des brevets

(11) Numéro de publication: **0 131 491**
**A1**

(12) **DEMANDE DE BREVET EUROPEEN**

(21) Numéro de dépôt: **84401204.7**

(22) Date de dépôt: **13.06.84**

(51) Int. Cl.⁴: **C 07 J 41/00**
**C 07 B 59/00, G 01 N 33/74**

(30) Priorité: **14.06.83 FR 8309812**
**20.06.83 FR 8310142**

(43) Date de publication de la demande:
**16.01.85 Bulletin 85/3**

(84) Etats contractants désignés:
**AT BE CH DE FR GB IT LI LU NL SE**

(71) Demandeur: **ROUSSEL-UCLAF**
**35, boulevard des Invalides**
**F-75007 Paris(FR)**

(72) Inventeur: **Jouquey, Alain**
**137, rue des Pyrénées**
**F-75020 Paris(FR)**

(72) Inventeur: **Salmon, Jean**
**8, rue du Général Castelnau**
**F-75015 Paris(FR)**

(72) Inventeur: **Mouren, Michel**
**184, avenue de Choisy**
**F-75013 Paris(FR)**

(72) Inventeur: **Touyer, Gaetan**
**54, rue Petit**
**F-75019 Paris(FR)**

(74) Mandataire: **Markovic, Borivoj et al,**
**ROUSSEL-UCLAF 111, route de Noisy Boîte Postale no 9**
**F-93230 Romainville(FR)**

(54) **Dérivés estradiéniques radioactifs marqués à l'iode, leur procédé et leurs intermédiaires de préparation et leur application pour l'étude et le dosage radioimmunologique de stéroides dans les fluides biologiques.**

(57) L'invention a pour objet des dérivés estradiéniques radioactifs marqués à l'iode de formule générale :

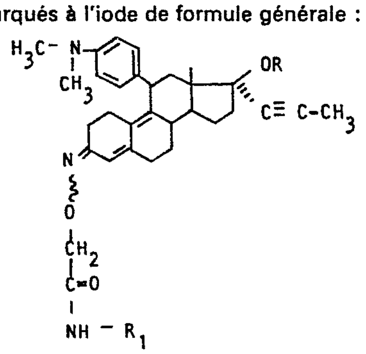

dans laquelle la ligne ondulée sur l'azote oximinique signifie que la fonction oxime peut être en position syn ou anti, formule dans laquelle R représente un hydrogène, un alcoyle (1 à 6 carbones) ou un acyle (1 à 12 carbones) et $R_1$ représente le reste d'un acide aminé $R_1NH_2$ ou le reste d'un dérivé de ce dernier, ce reste possédant un groupe accepteur d'iode et étant marqué à l'iode $^{125}$ ou $^{131}$, leur procédé et leurs intermédiaires de préparation et leur application pour l'étude et le dosage radioimmunologique de steroïdes dans les fluides biologiques

EP 0 131 491 A1

Dérivés estradiéniques radioactifs marqués à l'iode, leur procédé et leurs intermédiaires de préparation et leur application pour l'étude et le dosage radioimmunologique de stéroïdes dans les fluides biologiques.

La présente invention a pour objet de nouveaux dérivés estradiéniques radioactifs marqués à l'iode, leur procédé et leurs intermédiaires de préparation et leur application à l'étude et au dosage radioimmunilogique de stéroïdes dans les fluides biologiques.

Plus particulièrement, elle a pour objet des dérivés estradiéniques marqués à l'iode, sous forme de mélange d'isomères syn et anti ou sous forme d'isomères syn ou sous forme d'isomères anti, de formule générale (I) :

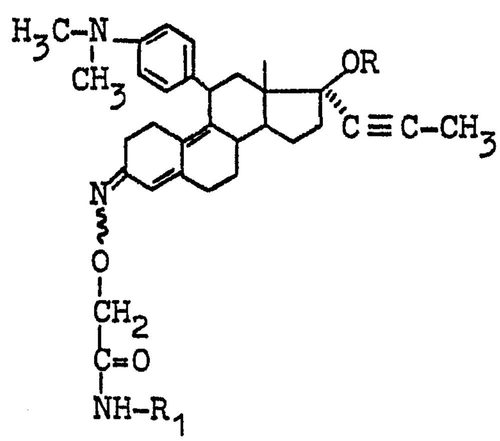

(I)

dans laquelle la ligne ondulée sur l'azote oximinique signifie que la fonction oxime peut être en position syn ou anti, formule dans laquelle

R représente un atome d'hydrogène, un groupement alcoyle ayant de 1 à 6 atomes de carbone ou un groupement acyle d'un acide carboxylique ayant de 1 à 12 atomes de carbone et $R_1$ représente le reste d'un acide aminé $R_1NH_2$ ou le reste d'un dérivé de ce dernier, ce reste étant accepteur d'iode et étant marqué à l'iode $^{125}$ ou $^{131}$.

Par reste d'un dérivé d'acide aminé, on entend de préférence le reste d'un dérivé décarboxylé d'un acide aminé ou le reste d'un ester d'alcoyle inférieur d'un acide aminé.

Parmi les valeurs de R, on peut citer :

a) les radicaux méthyle, éthyle, propyle, isopropyle, butyle, sec-butyle, tert-butyle, pentyle, iso-pentyle, sec-pentyle, tert-pentyle, néo-pentyle, hexyle, iso-hexyle, sec-hexyle, tert-hexyle ;

b) les radicaux formyle, acétyle, propionyle, butyryle, isobutyryle, valéryle, hexanoyle, benzoyle, caprylyle, méthoxy-carbonyle, éthoxy carbonyle.

L'invention a plus particulièrement pour objet les dérivés estradiéniques radioactifs marqués à l'iode de formule générale (I) dans laquelle $R_1$ représente le reste d'un acide aminé $R_1NH_2$ ou le reste d'un dérivé de ce dernier, choisi dans le groupement constitué par l'histidine, la tyrosine, —————— l'histamine, la tyramine et le tyrosinate de méthyle marqués à l'iode 125 ou 131.

Parmi les produits de formule générale (I), on citera notamment le 3-carboxyméthyloxime de 17β-hydroxy-11β-(4-diméthylaminophényl)-17α-(prop-1-ynyl)estra-4,9-dién-3-one sous forme de mélange d'isomère syn et d'isomère anti, sous forme d'isomère syn et sous forme d'isomère anti, couplé à l'$^{125}$I histamine (produit A) de formule :

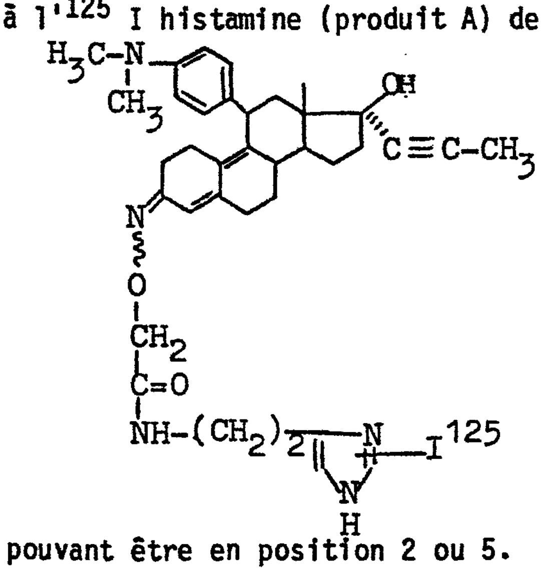

(Produit A)

l'iode pouvant être en position 2 ou 5.

L'invention a également pour objet un procédé de préparation des produits répondant à la formule générale (I) ci-dessus.

Ce procédé est caractérisé en ce que :

- on fait réagir un produit de formule générale (II) :

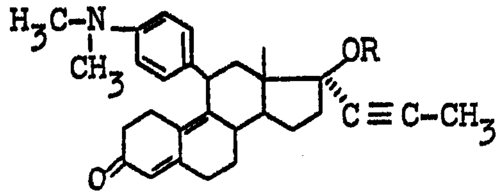

(II)

dans laquelle ____________ R a les significations précitées, ____________ avec un halogénure de carboxyméthoxylamine en présence d'une base et obtient un produit de formule (III) :

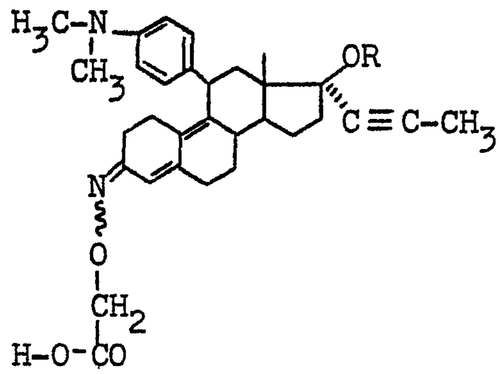

(III)

dans laquelle la ligne ondulée sur l'azote indique que ce produit est sous forme de mélange d'isomères syn et anti, puis

- fixe sur la fonction acide de ce produit un groupement activateur de la fonction carbonyle et obtient le produit de formule générale (IV) :

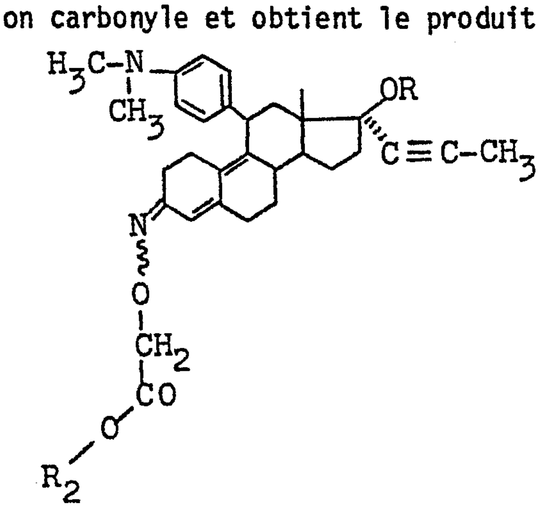

(IV)

dans laquelle $R_2$ représente un groupement activateur de la fonction carbonyle et la ligne ondulée sur l'azote indique que ce produit est sous forme de mélange d'isomères syn et anti,

- que l'on fait réagir avec un acide aminé $R_1NH_2$ possédant un groupe accepteur d'iode marqué à l'iode $^{125}$ ou $^{131}$ ou avec un

séparés, objet de l'invention, on peut procéder par exemple de la manière décrite ci-après :

on soumet un produit de formule générale (III), sous forme de mélange d'isomère syn et d'isomère anti, où R a les significations précitées, à l'action d'un agent d'estérification pour estérifier la fonction carboxyle, sépare l'isomère syn de l'isomère anti de l'ester résultant puis saponifie séparément ladite fonction ester de chacun des isomères syn et anti, obtient l'isomère syn et l'isomère anti des produits de formule générale (III), dans laquelle R garde les significations précitées et procède ensuite séparément, selon le procédé de l'invention, à la fixation sur la fonction acide libérée des isomères syn et anti, d'un groupement activateur de la fonction carbonyle, obtient les anhydrides mixtes correspondants, que l'on fait réagir avec un acide aminé accepteur d'iode marqué à l'iode $^{125}$ ou $^{131}$, ou avec un dérivé de cet acide, pour obtenir les produits de formule générale (I) correspondants sous formes d'isomères syn et anti séparés.

L'invention a également pour objet l'application des produits de formule générale (I) et notamment du 3-carboxyméthyloxime de 17β-hydroxy 11β-(4-diméthylaminophényl) 17α-(prop-1-ynyl) estra 4,9-dièn-3-one, sous forme de mélange d'isomère syn et d'isomère anti, sous forme d'isomère syn et sous forme d'isomère anti, couplé à l'$^{125}$I histamine (produit A), à l'étude et au dosage radioimmunologique de la 17β-hydroxy 11β-(4-diméthylaminophényl) 17α-(prop-1-ynyl) estra 4,9-dièn-3-one non radioactive (produit B) et de ses métabolites dans des fluides biologiques, chez l'homme ou chez l'animal.

L'invention a également pour objet à titre de produits industriels nouveaux, les produits de formule générale (III) et (IV) sous forme de mélange d'isomères syn et anti, ou sous forme d'isomère syn ou sous forme d'isomère anti :

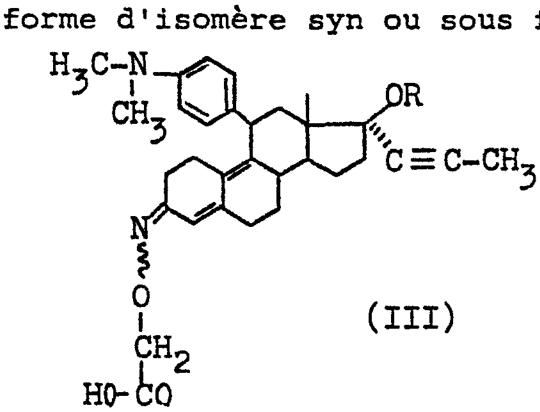
(III)

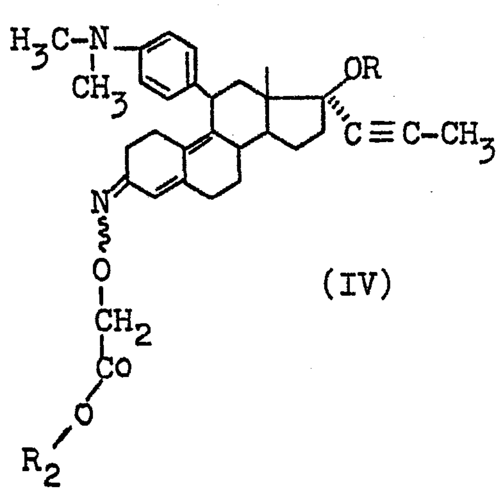
(IV)

dans lesquelles la ligne ondulée sur l'azote oximinique signifie que la fonction oxime peut être en position syn ou anti et dans laquelle R représente un atome d'hydrogène, un groupement alcoyle ayant de 1 à 6 atomes de carbone ou un groupement acyle d'un acide carboxylique ayant de 1 à 12 atomes de carbone et $R_2$ représente un groupement activateur de la fonction carbonyle.

Parmi ces produits, on citera les produits suivants :

- le 3-carboxyméthyloxime de 17β-hydroxy-11β-(4-diméthylaminophényl)-17α-(prop-1-ynyl)estra-4,9-diën-3-one sous forme de mélange d'isomères syn et anti, sous forme d'isomère syn et sous forme d'isomère anti,
- l'anhydride mixte de 3-carboxyméthyloxime de 17β-hydroxy-11β-(4-diméthylaminophényl)-17 α -(prop-1-ynyl)estra-4,9-diën-3-one avec le formiate d'isobutyle, sous forme de mélange d'isomères syn et anti, sous forme d'isomère syn et sous forme d'isomère anti :

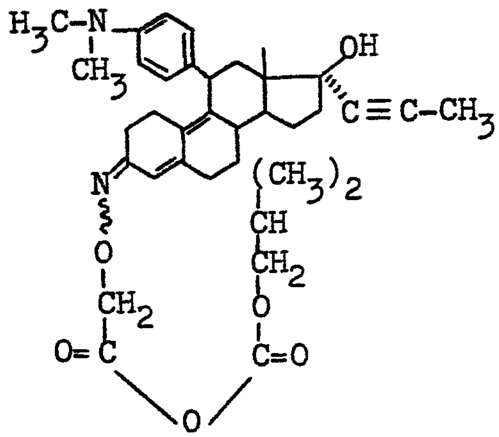

Les produits de formule générale (III) sont des produits de départ utiles pour la préparation d'antigènes, nécessaires également aux dosages radioimmunologiques du produit B précité et de ses métabolites.

Leur application en tant que telle fait également l'objet de la présente invention et est caractérisé en ce que l'on conjugue un de ces produits avec l'albumine sérique bovine (BSA) ou avec l'albumine sérique humaine (HSA) et obtient l'antigène cherché.

Dans des conditions préférentielles de mise en oeuvre, l'application des produits de formule générale (III), est caractérisée par les points suivants :

- on fait réagir un produit de formule générale (III) sous forme de mélange d'isomères syn et anti, sous forme d'isomère syn ou sous forme d'isomère anti :

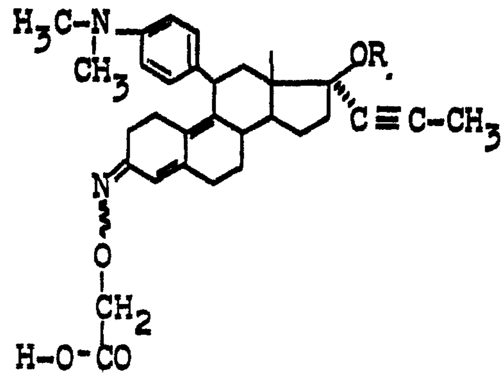

(III)

dans laquelle la ligne ondulée et R ont les significations précitées, en vue de l'activation de la fonction carbonyle, avec un halogénoformiate d'alcoyle, en présence d'une base tertiaire, en milieu anhydre et sous atmosphère interte, obtient l'anhydride mixte correspondant, de formule (IV) :

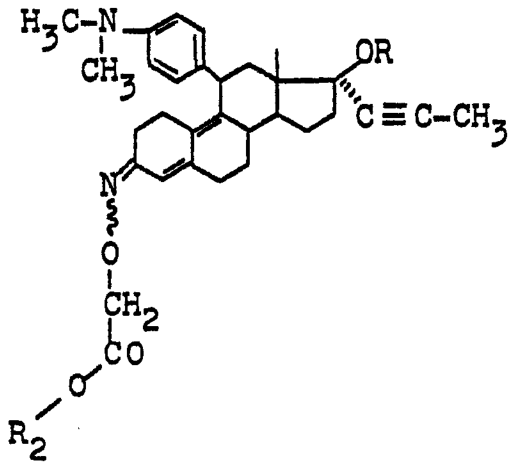

(IV)

dans laquelle R garde la signification précitée et $R_2$ représente un radical de formule -CO-O-alc, alc étant un radical alcoyle ayant au plus 6 atomes de carbone, que l'on conjugue avec l'albumine sérique bovine (BSA) ou humaine (HSA) et obtient l'antigène cherché.

Dans un mode d'exécution préférée, l'application des produits de formule générale (III) est encore caractérisée par les points suivants :

- l'halogénoformiate d'alcoyle est le chloroformiate d'isobutyle et on opère en présence de tri-n-butylamine et sous atmosphère inerte,
- on fait réagir l'anhydride mixte de formule générale (IV) avec l'albumine sérique bovine (BSA) ou humaine (HSA) en ayant préalablement dissout cette dernière dans un mélange eau-dioxane sous atmosphère inerte.

L'application selon l'invention permet d'obtenir au départ

du 3-carboxyméthyloxime de 17β-OR-11β-(4-diméthylaminophényl) 17α-(prop-1-ynyl) estra-4,9-dièn-3-one, sous forme de mélange d'isomère syn et d'isomère anti, sous forme d'isomère syn ou sous forme d'isomère anti, les antigènes de formules générales (V) et (VI) dans lesquelles R a les significations précitées :

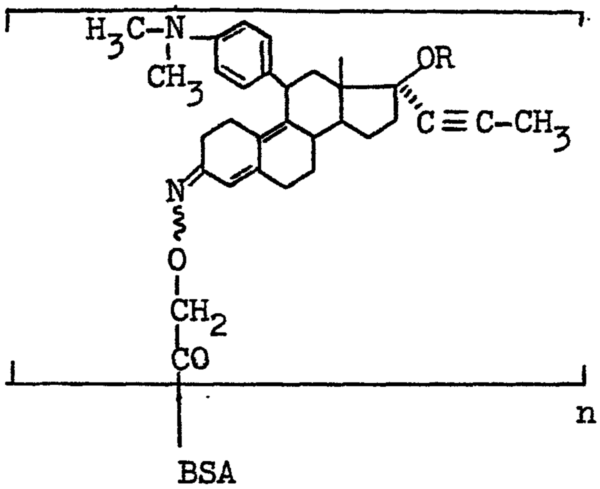

(V)

dans laquelle n=20 à 30 et la partie oxime de stéroïde est sous forme et sous forme de mélange d'isomères syn et anti, sous forme d'isomère syn ou sous forme d'isomère anti, et :

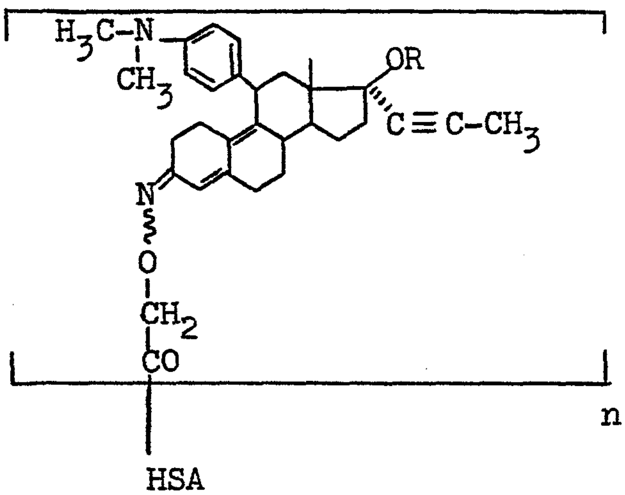

(VI)

dans laquelle n=20 à 30 et la partie oxime de stéroïde est sous forme de mélange d'isomères syn et anti, sous forme d'isomère syn ou sous forme d'isomère anti.

L'invention a également pour objet, à titre de produits industriels nouveaux, les antigènes obtenus lors de l'application décrite ci-dessus, et notamment les produits de formules générales (V) et (VI).

L'invention a également pour objet l'application des antigènes ci-dessus à la préparation des anticorps.

Les produits de formule générale (I), objet de l'invention et notamment le 3-carboxyméthyloxime de 17 β -hydroxy-11β-(4-diméthylaminophényl)-17 α -(prop-1-ynyl)-estra-4,9-dièn-3-one couplé à

l'($^{125}$I) histamine (produit A), sous forme de mélange d'isomère syn et d'isomère anti, sous forme d'isomère syn ou sous forme d'isomère anti, sont utilisés comme déjà dit lors des études et lors du dosage radioimmunologique de la 17$\beta$-hydroxy-11$\beta$-(4-diméthylaminophényl)-17$\alpha$-(prop-1-ynyl)-estra-4,9-dièn-3-one non radioactive (produit B) et de ses métabolites dans des fluides biologiques chez l'homme ou chez l'animal.

Le produit A, après son administration, peut être suivi dans son évolution et dans son comportement au niveau des fluides biologiques chez l'homme et chez l'animal.

Lors de ces études, le produit A permet notamment un dosage spécifique aisé de quantités de l'ordre de quelques dizaines de picogrammes par ml de fluide biologique, sans que l'on soit obligé de recourir à des méthodes d'isolement et de purification par chromatographie, avant de procéder au dosage proprement dit.

Pour étudier et doser par des méthodes radioimmunologiques la 17$\beta$-hydroxy-11$\beta$-(4-diméthylaminophényl)-17$\alpha$-(prop-1-ynyl)-estra-4,9-dièn-3-one non radioactive (produit B), on prépare comme déjà dit à partir de ce dernier, des antigènes avec l'albumine sérique bovine (BSA) ou avec l'albumine sérique humaine (HSA).

Les antigènes résultant de formules (V) et (VI), également objets de l'invention peuvent servir au développement d'anticorps lorsqu'ils sont injectés chez l'animal en présence d'un adjuvant. Ils permettent ainsi d'obtenir des serums contenant des anticorps.

Ces anticorps servent ensuite de récepteurs de produits radioactifs et/ou de produits non-radioactifs, à savoir de récepteurs de produits de formule générale (I) et notamment du 3-carboxyméthyloxime de 17$\beta$-hydroxy-11$\beta$-(4-diméthylaminophényl)-17$\alpha$-(prop-1-ynyl)-estra-4,9-dièn-3-one sous forme de mélange d'isomère syn et d'isomère anti, sous forme d'isomère syn et sous forme d'isomère anti, couplé à l'($^{125}$I) histamine (produit A), ainsi que de la 17$\beta$-hydroxy 11$\beta$-(4-diméthylaminophényl) 17$\alpha$-(prop-1-ynyl) estra 4,9-dièn-3-one non radioactive (produit B) et de ses métabolites.

La présence de ces anticorps est mise en évidence au moyen d'un produit de formule générale (I) et notamment au moyen du 3-carboxyméthyloxime de 17$\beta$-hydroxy-11$\beta$-(4-diméthylaminophényl)-17$\alpha$-(prop-1-ynyl)-estra-4,9-dièn-3-one sous forme de mélange d'isomère syn et d'isomère anti, sous forme d'isomère syn et sous forme d'isomère anti, couplé à l'($^{125}$I) histamine radioactif (produit A).

On procède ensuite au dosage du produit B ou de ses métabolites selon des méthodes radioimmunologiques conventionnelles, telles que celle décrites par :

- S.A. BERGSON et R.S. YALOW, HORMONE 4, p. 557 (1964) et
- G.E. ABRAHAM J. of CHEM. ENDOCRINAL METAB., 29, p. 866 (1969).

Les exemples suivants illustrent l'invention sans toutefois la limiter.

EXEMPLE 1 : 3-carboxyméthyloxime de 17 β -hydroxy-11β-(4-diméthylaminophényl)17 α -(prop-1-ynyl)-estra-4,9-dièn-3-one couplé à l'($^{125}$I) histamine.

STADE A :3-carboxyméthyloxime de 17 β -hydroxy-11β-(4-diméthylaminophényl)-17α (prop-1-ynyl)-estra-4,9-dièn-3-one.

A une solution de 1 g de 17β-hydroxy-11β-(4-diméthylaminophényl)-17 α -(prop-1-ynyl)-estra-4,9-dièn-3-one dans 35 ml d'éthanol, on ajoute 665 mg d'hemihydrochlorure de carboxyméthoxylamine, soumet le mélange réactionnel à l'agitation pendant une heure environ à température ambiante, additionne de 1,6 ml de soude N, puis concentre sous vide, reprend le résidu par le chloroforme, essore, évapore le filtrat à sec, le chromatographie sur silice (éluants chloroforme/méthanol 7:3), isole le produit cherché que l'on empâte par l'éther isopropylique, essore et sèche sous vide pour obtenir 1,13 g du dit produit cherché sous forme de mélange d'isomères syn et anti. La chromatographie sur couche mince de silice, en éluant au mélange chloroforme/méthanol (8:2) donne Rf=0,45.

STADE B :3-carboxyméthyloxime de 17 β -hydroxy-11β-(4-diméthylaminophényl)-17 α -(prop-1-ynyl)-estra-4,9-dièn-3-one couplé à l'($^{125}$I) histamine sous forme de mélange d'isomères syn et anti.

Préparation d'anhydride mixte de 3-carboxyméthyloxime de 17β-hydroxy-11β -(4-diméthylaminophényl)-17 α -(prop-1-ynyl)-estra-4,9-dièn-3-one sous forme de mélange d'isomères syn et anti, avec le formiate d'isobutyle.

On dissout sous agitation et sous atmosphère inerte 3,5 mg de 3-carboxyméthyloxime de 17 β -hydroxy-11 β -(4-diméthylaminophényl)-17α -(prop-1-ynyl-estra-4,9-dièn-3-one préparé au stade précédent dans 50μl de dioxane, ajoute 10μl de mélange tri-n-butylamine/tétrahydrofuranne (1:5) puis 10μl de mélange chloroformiate d'isobutyle/tétrahydrofuranne (1:10), maintient ce mélange réactionnel sous agitation pendant une demi-heure à 4°C environ, puis additionne de 3,4 ml de tétrahydrofuranne et obtient une solution du produit cherché, que l'on utilise telle qu'elle pour le stade suivant de la synthèse.

Iodation de l'histamine.

A 10µl d'une solution d'histamine 2 mM dans une solution tamponnée de phosphate de sodium 0,5 M de pH8, on ajoute successivement 1 mCi de iodure $^{125}$ de sodium et 50µg de chloramine T dans 10µl d'eau distillée. On agite le mélange réactionnel pendant 90 secondes environ, ajoute 300µg de métabisulfite de sodium dissout dans 10µl d'eau distillée et obtient une solution aqueuse de produit cherché que l'on utilise telle que pour la suite de la synthèse (chromatographie sur couche mince de silice, système de solvant méthanol/triéthylamine (98:2) : Rf = 0,1.

Condensation

On ajoute, sous atmosphère inerte, à la solution d'histamine iodée obtenue ci-dessus, 50µl de solution d'anhydride mixte, précédemment préparée, agite et abandonne au repos à environ 4°C sous atmosphère inerte pendant environ 1 heure et demie.

On dilue ensuite le mélange réactionnel par 400µl d'une solution de bicarbonate de sodium 0,1 M, extrait par 1,6 ml de chlorure de méthylène, sépare la phase organique et l'évapore à sec sous atmosphère inerte. On reprend le résidu par 250µl d'acétate d'éthyle, purifie le produit par chromatographie liquide haute performance avec élution par le système chloroforme/méthanol (98,5:1,5). On isole et évapore à sec la solution correspondant à 2 pics grossièrement séparés, sous atmosphère inerte. Les résidus sont repris par le méthanol de façon à obtenir une concentration en produit radioactif d'une activité de 25µCi/ml. On obtient environ 100µCi de produit en tout.

L'ensemble de la préparation du produit iodé est fait en lumière inactinique.

La chromatographie sur couche mince de silice avec l'élution par le système cyclohexane/éthanol/triéthylamine (70:30:1) donne Rf=0,28.

EXEMPLE 2 : antigène à partir de 3-carboxyméthyloxime de 17β-hydroxy-11-β(4-diméthylaminophényl)-17α-(prop-1-ynyl)-estra-4,9-diên-3-one sous forme de mélange d'isomères syn et anti et d'albumine sérique bovine(BSA).

STADE A : Anhydride mixte de 3-carboxyméthyloxime de 17β-hydroxy-11β-(4-diméthylaminophényl)-17α-(prop-1-ynyl)-estra-4,9-diên-3-one sous forme de mélange d'isomères syn et anti avec le formiate d'isobutyle.

On dissout sous agitation et sous atmosphère inerte 63 mg de 3-carboxyméthyloxime de 17β-hydroxy-11β-(4-diméthylaminophényl)-17α-(prop-1-ynyl)-estra-4,9-diên-3-one, préparé au stade A de l'exemple 1 dans 1,2 ml de dioxane, ajoute 57,5µl de tri-n-butylamine, puis abaisse la température vers 13°C, ajoute 16µl de chloroformiate d'isobutyle,

maintient ce mélange réactionnel sous agitation à la températurede 13°C environ pendant une demi-heure. On obtient une solution du produit cherché, que l'on utilise telle quelle pour le stade suivant de la synthèse.

STADE B :Dissolution de l'albumine sérique bovine.

On introduit à 4°C sous agitation et sous atmosphère inerte 175 mg d'albumine sérique bovine dans 0,5 ml d'eau, maintient sous agitation pendant 20 mn environ, rajoute successivement après la dissolution 3 ml de dioxane et 170$\mu$l de NaOH N.

STADE C :Conjugaison.

On verse lentement dans la solution d'albumine sérique bovine obtenue au stade B, la solution d'anhydride mixte obtenue au stade A, amène le pH à 8,9 à l'aide d'acide chlorhydrique N et agite le mélange réactionnel pendant 5 heures à 10°C environ. On sépare ensuite la phase organique du précipité formé, la diluepar 30 ml d'eau distillée, amène le pH à 4,1 par l'addition d'acide chlorhydrique N, sépare le précipité formé, le reprend par 25 ml d'une solution aqueuse debicarbonate de sodium à 1 %. La solution résultante est laissée au repos , puis soumise à l'utrafiltration. On épuise ensuite la solution des molécules de poids moléculaire <10-15000 par 510 ml d'eau distillée puis ajuste le volume final à 17 ml. On lyophilise cette solution pendant 30 heures environ et obtient 169 mg d'antigène cherché.

Analyse

Spectre UV

- dans eau : max 265 nm $E^1_1$=53
  max 290 nm $E^1_1$=62
- dans HCl N/10 : max 290 nm $E_1^1$=61

Dichroïsme circulaire

- dans eau : max 222 nm $\Delta E^1_1$=-0,305
  max 260 nm $\Delta E^1_1$=-0,070
  max 289 nm $\Delta E^1_1$=-0,050
- dans HCl N/10 : max 222nm $\Delta E^1_1$=-0,250
  max 290 nm $\Delta E^1_1$:+0,027

Ce produit contient 20 à 22 groupes de stéroïde liés par mole de protéïne.

EXEMPLE 3 : antigène à partir de 3-carboxyméthyloxime de 17$\beta$-hydroxy-11$\beta$-(4-diméthylaminophényl)-17$\alpha$-(prop-1-ynyl)-estra-4,9-diên-3-one sous forme de mélange d'isomères syn et anti et d'albumine sérique humaine (HSA).

En procédant comme décrit dans le stade C de l'exemple 2,mais en

partant de la solution d'anhydride mixte du stade A et d'une solution d'albumine sérique humaine préparée selon la méthode décrite au stade B, on obtient 180 mg d'antigène cherché.

Ce produit contient 34 à 36 groupes de stéroïde liés par mole de protéine.

<u>Analyse</u>

<u>Spectre UV</u>

- dans eau : max 265 nm $E^1_1=79$
  max 291 nm $E^1_1=94$
- dans HCl N/10 : max 290 nm $E^1_1=93$

<u>Dichroïsme circulaire</u>

- dans eau : infl. 218 nm $\Delta E^1_1=-0,308$
  max 259 nm $\Delta E^1_1=-0,115$
  max 288 nm $\Delta E^1_1=+0,085$
- dans HCl N/10 : max 223 nm $\Delta E^1_1=-0,300$
  max 283 nm $\Delta E^1_1=+0,037$

Revendications :

1) Produits estradiéniques marqués à l'iode 125 ou 131 sous forme de mélange d'isomères syn et anti ou sous forme d'isomères syn ou anti de formule générale (I) :

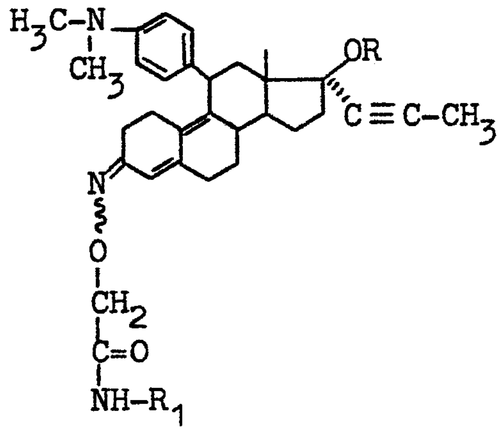

(I)

dans laquelle la ligne ondulée sur l'azote oximinique signifie que la fonction oxime peut être en position syn ou anti, formule dans laquelle R représente un atome d'hydrogène, un groupement alcoyle ayant de 1 à 6 atomes de carbone ou un groupement acyle d'un acide carboxylique ayant de 1 à 12 atomes de carbone et $R_1$ représente le reste d'un acide aminé $R_1$ $NH_2$ ou le reste d'un dérivé de ce dernier, ce reste possédant un groupe accepteur d'iode et étant marqué à l'iode $^{125}$ ou $^{131}$.

2) Produits estradiéniques radioactifs marqués à l'iode de formule générale (I) selon la revendication 1 dans laquelle $R_1$ représente le reste d'un acide aminé $R_1$ $NH_2$ou le reste d'un dérivé de ce dernier, choisi dans le groupe constitué par l'histidine, la tyrosine, ~~——————~~ l'histamine, la tyramine et le tyrosinate de méthyle, marqués à l'iode $^{125}$ ou $^{131}$.

3) Le 3-carboxyméthyloxime de 17β -hydroxy-11β-(4-diméthylaminophényl)-17α -(prop-1-ynyl)-estra-4,9-diên-3-one, sous forme de mélange d'isomère syn et d'isomère anti, sous forme d'isomère syn ou sous forme d'isomère anti, couplé à l'($^{125}$I) histamine, l'iode pouvant être en position 2 ou 5, de formule :

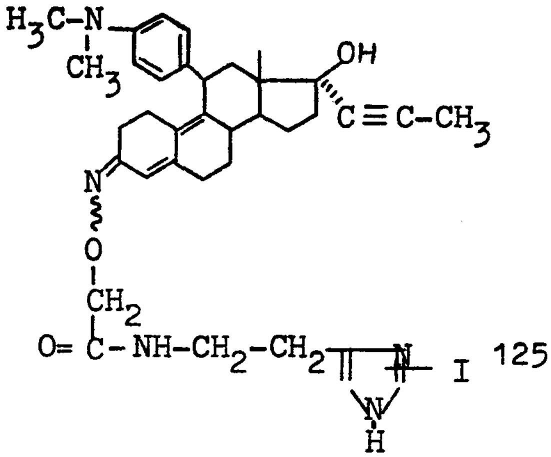

4) Procédé de préparation des produits définis aux revendications 1 à 3, caractérisé en ce que :

- on fait réagir un produit de formule générale (II) :

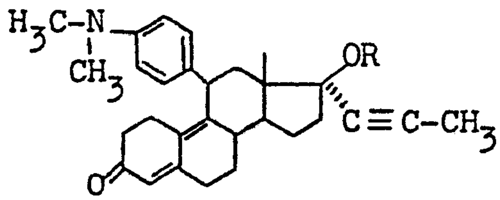

(II)

dans laquelle ________ R a les significations indiquées à la revendication 1, avec un halogénure de carboxyméthoxylamine en présence d'une base et obtient un produit de formule (III) :

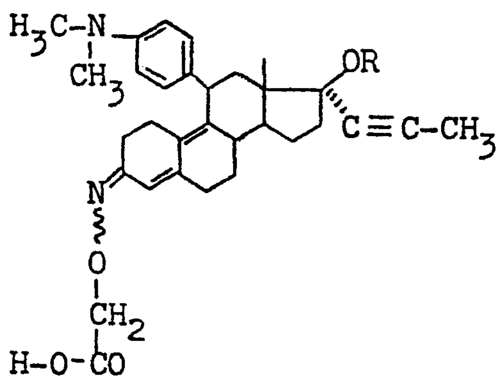

(III)

dans laquelle la ligne ondulée sur l'azote indique que ce produit est sous forme de mélange d'isomères syn et anti,puis

- fixe sur la fonction acide de ce produit un groupement activateur de la fonction carbonyle et obtient le produit de formule générale (IV) :

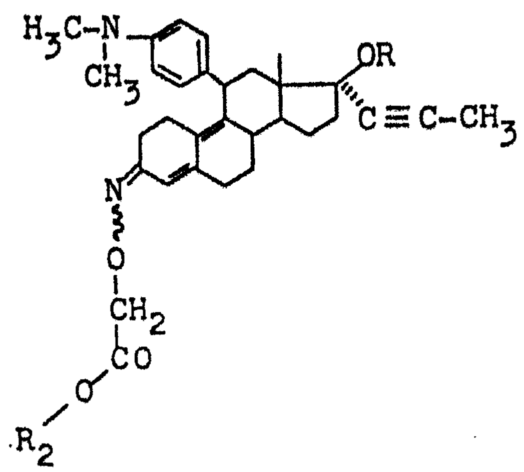

(IV)

dans laquelle $R_2$ représente un groupement activateur de la fonction carbonyle et la ligne ondulée sur l'azote indique que ce produit est sous forme de mélange d'isomères syn et anti,

- que l'on fait réagir avec un acide aminé $R_1-NH_2$ possédant un groupe accepteur d'iode marqué à l'iode $^{125}$ ou $^{131}$ ou avec un dérivé de cet acide et obtient le produit cherché de formule générale (I), que l'on isole sous forme de mélange d'isomères syn et anti ou que l'on sépare, si désiré, en ses isomères, puis, le cas échéant, éthérifie ou estérifie la fonction hydroxyle en 17.

5) Procédé selon la revendication 4, caractérisé en ce que l'acide aminé accepteur d'iode ou le dérivé d'acide aminé est choisi dans le groupe constitué par l'histidine, la tyrosine, l'histamine, la tyramine et le tyrosinate de méthyle.

6) Procédé selon la revendication 4 ou 5, caractérisé en ce que :

- l'halogénure de carboxyméthoxylamine est l'hémihydrochlorure de carboxyméthoxylamine et on opère sous atmosphère inerte et en présence de soude,
- on fixe sur la fonction acide un groupement activateur de la fonction carbonyle en faisant agir un halogénoformiate d'alcoyle en présence d'une base tertiaire, en milieu anhydre et sous atmosphère inerte,

7) Procédé selon la revendication 6, caractérisé en ce que l'halogénoformiate d'alcoyle est le chloroformiate d'isobutyle et on opère en présence de tri-n-butylamine.

8) Procédé suivant l'une quelconque des revendications 4 à 7, caractérisé en ce que l'acide aminé que l'on fait réagir avec le produit de formule générale (IV) dans laquelle $R_2$ représente un groupement activateur de la fonction carbonyle, est l'histamine marquée à l'iode $^{125}$ ou $^{131}$ et l'on opère sous atmosphère inerte.

9) Procédé suivant l'une quelconque des revendications 4 à 8,

caractérisé en ce que le produit de départ étant la 17β-hydroxy-11β-(4-diméthylaminophényl)-17α-(prop-1-ynyl)-estra-4,9-dièn-3-one, on obtient le produit de formule :

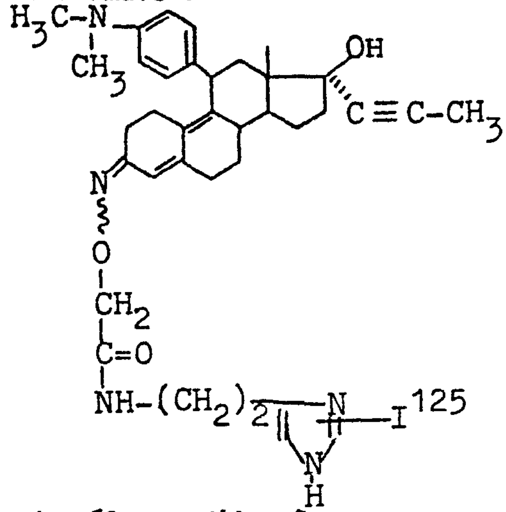

sous forme de mélange d'isomères syn et anti, ou sous forme d'isomère syn ou sous forme d'isomère anti.

10) Application des produits tels que décrits aux revendications 1 à 3, à l'étude et au dosage radioimmunologique de la 17β-hydroxy 11β-(4-diméthylaminophényl) 17α-(prop-1-ynyl) estra 4,9-dièn-3-one non radioactive et de ses métabolites, dans les fluides biologiques, chez l'homme ou chez l'animal.

11) Les produits de formule (I) tels que définis à l'une quelconque des revendications 1 à 3, pour leur utilisation chez l'homme ou l'animal, pour l'étude et le dosage radioimmunologique de la 17β-hydroxy 11β-(4-diméthylaminophényl) 17α-(prop-1-ynyl) estra 4,9-dièn-3-one non radioactive et de ses métabolites dans les fluides biologiques.

12) A titre de produits industriels nouveaux, les produits de formules générales (III) et (IV), sous forme de mélange d'isomères syn et anti, sous forme d'isomère syn ou sous forme d'isomère anti :

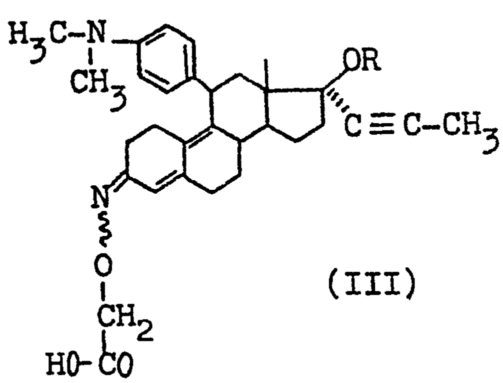

(III)

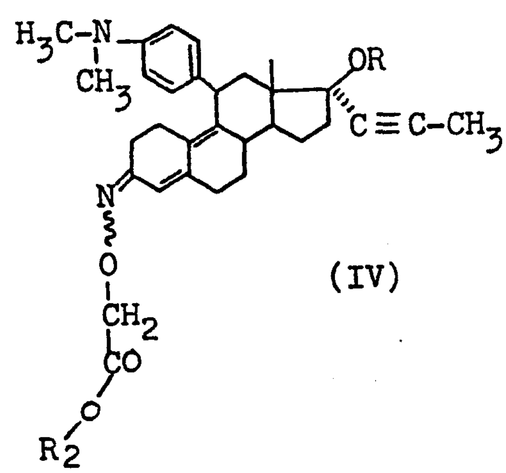

(IV)

dans lesquelles la ligne ondulée sur l'azote oximinique signifie que la fonction oxime peut être en position syn ou anti et dans laquelle R représente un atome d'hydrogène, un groupement alcoyle ayant de 1 à 6 atomes de carbone ou un groupement acyle d'un acide carboxylique ayant de 1 à 12 atomes de carbone et $R_2$ représente un groupement activateur de la fonction carbonyle.

13) Produits selon la revendication 12, à savoir :

- le 3-carboxyméthyloxime de 17β-hydroxy-11β-(4-diméthylaminophényl)-17α-(prop-1-ynyl)-estra-4,9-diên-3-one sous forme de mélange d'isomères syn et anti, sous forme d'isomère syn et sous forme d'isomère anti,
- l'anhydride mixte de 3-carboxyméthyloxime de 17β-hydroxy-11β-(4-diméthylaminophényl)-17 α -(prop-1-ynyl)-estra-4,9-diên-3-one avec le formiate d'isobutyle, sous forme de mélange d'isomères syn et anti, sous forme d'isomère syn et sous forme d'isomère anti :

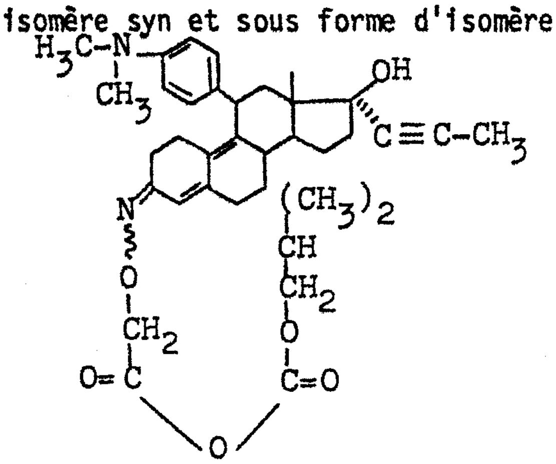

14) Application des produits de formule générale (III) tels que définis à la revendication 12 ou 13, à la préparation d'antigènes, caractérisée en ce que l'on conjugue un de ces produits avec de l'albumine sérique bovine (BSA) ou avec de l'albumine sérique humaine (HSA) et obtient l'antigène cherché.

15) Application selon la revendication 14, caractérisée en ce que l'on fait réagir un produit de formule générale (III), sous forme de mélange d'isomères syn et anti, sous forme d'isomère syn ou sous forme d'isomère anti :

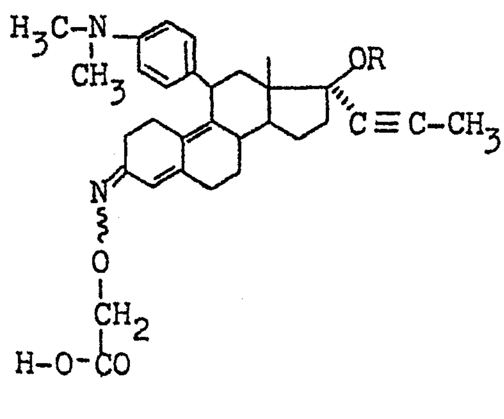

(III)

dans laquelle la ligne ondulée et R ont les significations précitées, en vue de l'activation de la fonction carbonyle, avec un halogénoformiate d'alcoyle, en présence d'une base tertiaire, en milieu anhydre et sous atmosphère inerte, obtient l'anhydride mixte correspondant, de formule (IV) :

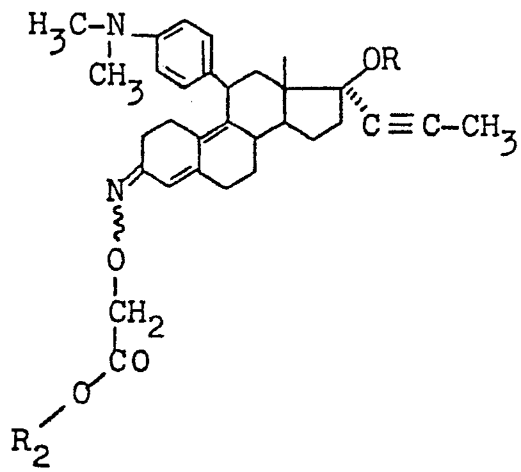

(IV)

dans laquelle R garde la signification précitée et $R_2$ représente un radical de formule -CO-O-alc, alc étant un radical alcoyle ayant au plus 6 atomes de carbone, que l'on conjugue avec l'albumine sérique bovine (BSA) ou humaine (HSA) et obtient l'antigène cherché.

16) Application selon la revendication 15, caractérisée en ce que :

- l'halogénoformiate d'alcoyle est le chloroformiate d'isobutyle et l'on opère en présence de tri-n-butylamine et sous atmosphère inerte ;
- on fait réagir l'anhydride mixte de formule générale (IV) avec l'albumine sérique bovine (BSA) ou humaine (HSA) en ayant préalablement dissout cette dernière dans un mélange eau-dioxane sous atmosphère inerte.

17) Antigènes constitués par la conjugaison d'un produit de formule générale (III) tel que défini à la revendication 12, avec de l'albumine sérique bovine (BSA) ou avec de l'albumine sérique humaine (HSA), à savoir les antigènes de formule (V) et (VI) dans lesquelles R a les significations données à la revendication 1 :

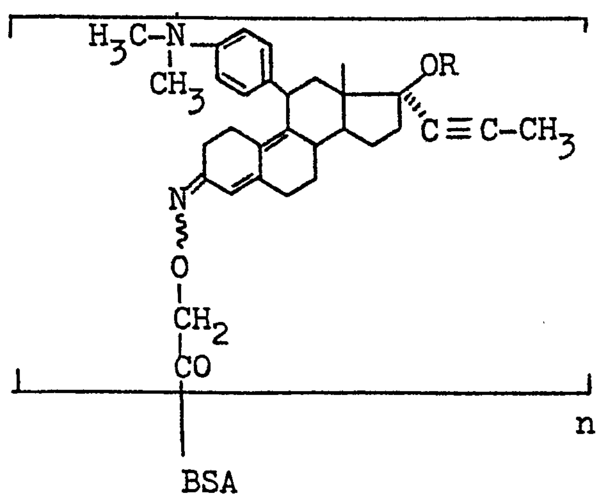

(V)

dans laquelle n=20 à 30 et la partie oxime de stéroïde est sous forme d'isomère syn, d'isomère anti ou sous forme de mélange d'isomères syn et anti et :

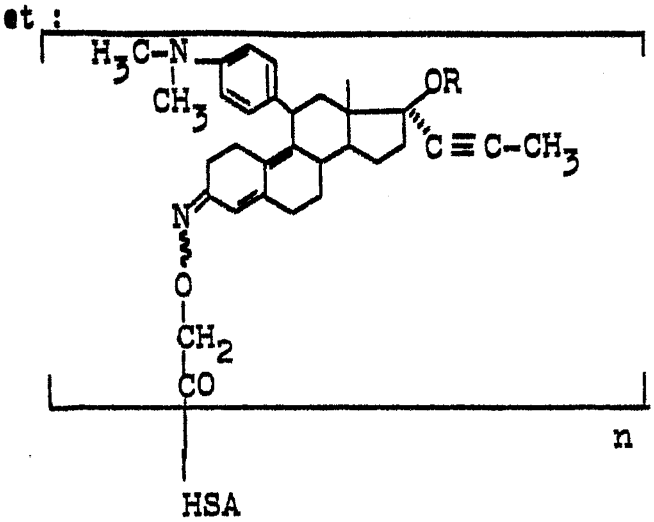

(VI)

dans laquelle n=20 à 30 et la partie oxime de stéroïde est sous forme d'isomère syn, sous forme d'isomère anti ou sous forme de mélange d'isomères syn et anti.

18) Application des antigènes selon la revendication 17, à la préparation des anticorps.

**Pour Autriche :**

0131491

Revendications :

1) Procédé de préparation des produits estradiéniques marqués à l'iode 125 ou 131 sous forme de mélange d'isomères syn et anti ou sous forme d'isomères syn ou anti de formule générale (I) :

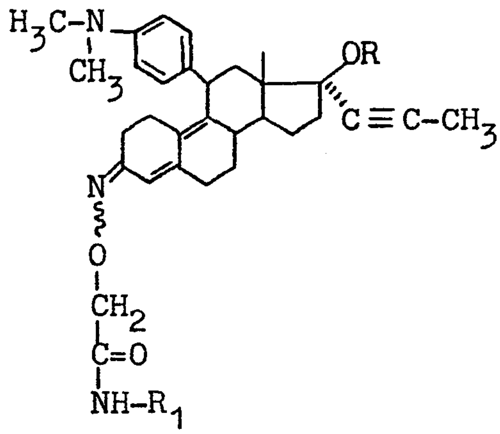

(I)

dans laquelle la ligne ondulée sur l'azote oximinique signifie que la fonction oxime peut être en position syn ou anti, formule dans laquelle R représente un atome d'hydrogène, un groupement alcoyle ayant de 1 à 6 atomes de carbone ou un groupement acyle d'un acide carboxylique ayant de 1 à 12 atomes de carbone et $R_1$ représente le reste d'un acide aminé $R_1$ $NH_2$ ou le reste d'un dérivé de ce dernier, ce reste possédant un groupe accepteur d'iode et étant marqué à l'iode 125 ou 131, caractérisé en ce que :

- on fait réagir un produit de formule générale (II) :

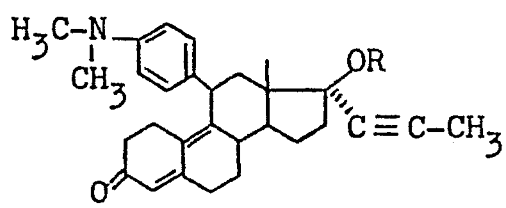

(II)

dans laquelle ―――――――― R a les significations indiquées à la précédemment , avec un halogénure de carboxyméthoxylamine en présence d'une base et obtient un produit de formule (III) :

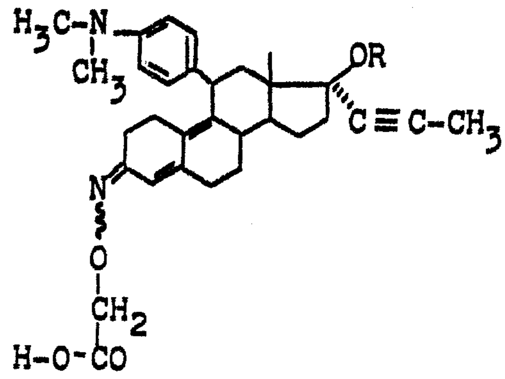

(III)

dans laquelle la ligne ondulée sur l'azote indique que ce produit est sous forme de mélange d'isomères syn et anti, puis

- fixe sur la fonction acide de ce produit un groupement activateur de la fonction carbonyle et obtient le produit de formule générale (IV) :

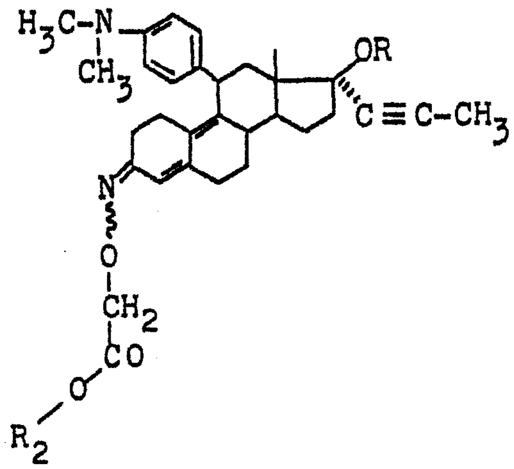

(IV)

dans laquelle $R_2$ représente un groupement activateur de la fonction carbonyle et la ligne ondulée sur l'azote indique que ce produit est sous forme de mélange d'isomères syn et anti,

- que l'on fait réagir avec un acide aminé $R_1-NH_2$ possédant un groupe accepteur d'iode marqué à l'iode $^{125}$ ou $^{131}$ ou avec un dérivé de cet acide et obtient le produit cherché de formule générale (I), que l'on isole sous forme de mélange d'isomères syn et anti ou que l'on sépare, si désiré, en ses isomères, puis, le cas échéant, éthérifie ou estérifie la fonction hydroxyle en 17.

2) Procédé selon la revendication 1, caractérisé en ce que l'acide aminé accepteur d'iode ou le dérivé d'acide aminé est choisi dans le groupe constitué par l'histidine, la tyrosine, l'histamine, la tyramine et le tyrosinate de méthyle.

3) Procédé selon la revendication 1 ou 2, caractérisé en ce que :

- l'halogénure de carboxyméthoxylamine est l'hémihydrochlorure de

carboxyméthoxylamine et on opère sous atmosphère inerte et en présence de soude,

- on fixe sur la fonction acide un groupement activateur de la fonction carbonyle en faisant agir un halogénoformiate d'alcoyle en présence d'une base tertiaire, en milieu anhydre et sous atmosphère inerte,

4) Procédé selon la revendication 3, caractérisé en ce que l'halogénoformiate d'alcoyle est le chloroformiate d'isobutyle et on opère en présence de tri-n-butylamine.

5) Procédé suivant l'une quelconque des revendications 1 à 4, caractérisé en ce que l'acide aminé ———— que l'on fait réagir avec le produit de formule générale (IV) dans laquelle $R_2$ représente un groupement activateur de la fonction carbonyle, est l'histamine marquée à l'iode $^{125}$ ou $^{131}$ ———— et l'on opère sous atmosphère inerte.

6) Procédé suivant l'une quelconque des revendications 1 à 5, caractérisé en ce que le produit de départ étant la 17β-hydroxy-11β-(4-diméthylaminophényl)-17α-(prop-1-ynyl)-estra-4,9-diën-3-one, on obtient le produit de formule :

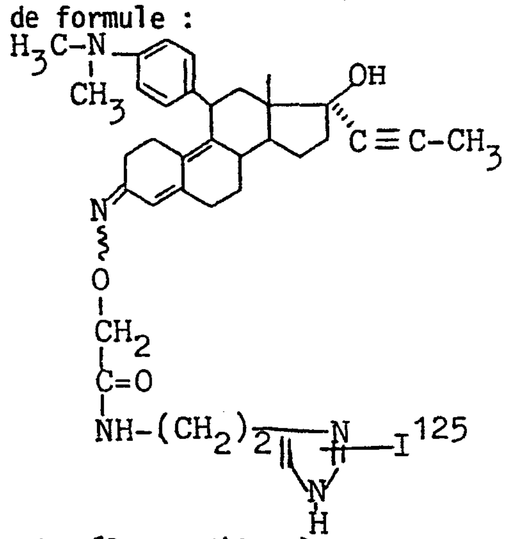

sous forme de mélange d'isomères syn et anti, ou sous forme d'isomère syn ou sous forme d'isomère anti.

7) Application des produits de formule (I) telle que définie à la revendication 1, à l'étude et au dosage radioimmunologique de la 17β-hydroxy 11β-(4-diméthylaminophényl) 17α-(prop-1-ynyl) estra 4,9-diën-3-one non radioactive et de ses métabolites, dans les fluides biologiques, chez l'homme ou chez l'animal.

8) Application des produits de formule générale (III) tels que définis à la revendication 4 ————, à la préparation d'antigènes, caractérisée en ce que l'on conjugue un de ces produits avec de l'albumine sérique bovine (BSA) ou avec de l'albumine sérique humaine (HSA) et obtient l'antigène cherché.

9 ) Application selon la revendication 8, caractérisée en ce que l'on fait réagir un produit de formule générale (III), sous forme de mélange d'isomères syn et anti, sous forme d'isomère syn ou sous forme d'isomère anti :

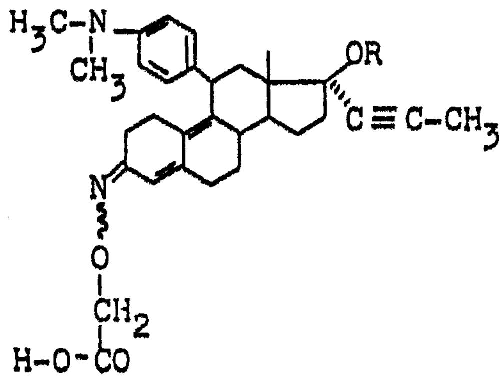

(III)

dans laquelle la ligne ondulée et R ont les significations précitées, en vue de l'activation de la fonction carbonyle, avec un halogénoformiate d'alcoyle, en présence d'une base tertiaire, en milieu anhydre et sous atmosphère inerte, obtient l'anhydride mixte correspondant, de formule (IV) :

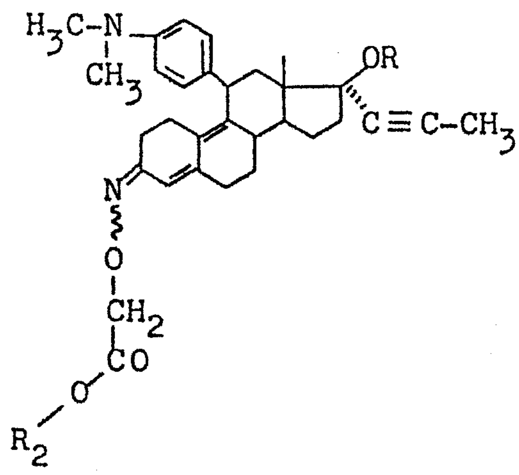

(IV)

dans laquelle R garde la signification précitée et $R_2$ représente un radical de formule -CO-O-alc, alc étant un radical alcoyle ayant au plus 6 atomes de carbone, que l'on conjugue avec l'albumine sérique bovine (BSA) ou humaine (HSA) et obtient l'antigène cherché.

10) Application selon la revendication 9, caractérisée en ce que :

- l'halogénoformiate d'alcoyle est le chloroformiate d'isobutyle et l'on opère en présence de tri-n-butylamine et sous atmosphère inerte ;
- on fait réagir l'anhydride mixte de formule générale (IV) avec l'albumine sérique bovine (BSA) ou humaine (HSA) en ayant préalablement dissout cette dernière dans un mélange eau-dioxane sous atmosphère inerte.

Office européen des brevets

**RAPPORT DE RECHERCHE EUROPEENNE**

0131491

Numéro de la demande

EP 84 40 1204

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | EP-A-0 057 115 (ROUSSEL-UCLAF) * revendication 1; pages 32-33, exemple 4; page 60, lignes 16-21 * | 1,11, 12 | C 07 J 41/00<br>C 07 B 23/00<br>G 01 N 33/74 |
| A | FR-A-2 397 426 (BECKMAN INSTRUMENTS) * en entier * | 1-3,10 ,11 | |
| A | US-A-4 358 435 (WILSON R. SLAUNWHITE) * colonnes 6-10 * | 1,3,10 ,11 | |

DOMAINES TECHNIQUES RECHERCHES (Int. Cl. 3)

C 07 J 41/00

Le présent rapport de recherche a été établi pour toutes les revendications

| Lieu de la recherche | Date d'achèvement de la recherche | Examinateur |
|---|---|---|
| LA HAYE | 25-09-1984 | HENRY J.C. |

CATEGORIE DES DOCUMENTS CITES

- X : particulièrement pertinent à lui seul
- Y : particulièrement pertinent en combinaison avec un autre document de la même catégorie
- A : arrière-plan technologique
- O : divulgation non-écrite
- P : document intercalaire
- T : théorie ou principe à la base de l'invention
- E : document de brevet antérieur, mais publié à la date de dépôt ou après cette date
- D : cité dans la demande
- L : cité pour d'autres raisons
- & : membre de la même famille, document correspondant

OEB Form 1503.03.82